# EUROPÄISCHE PATENTSCHRIFT

(11) **EP 0 762 846 B1**
(45) Veröffentlichungstag und Bekanntmachung des Hinweises auf die Patenterteilung: **04.03.1998**
(21) Anmeldenummer: 95920746.5
(22) Anmeldetag: 01.06.1995
(51) Int. Cl.: A61B 3/11, A61B 3/113, A61B 3/12

(54) **VORRICHTUNG ZUM MESSEN DER PUPILLENREAKTION EINES AUGES**
INSTRUMENT FOR MEASURING EYE-PUPIL REACTION
SYSTEME PERMETTANT DE MESURER LES REACTIONS D'UN OEIL AU NIVEAU PUPILLAIRE

(30) Priorität: 03.06.1994 DE 4419489
(43) Veröffentlichungstag der Anmeldung: 19.03.1997
(73) Patentinhaber: AMTECH GESELLSCHAFT FÜR ANGEWANDTE MICROCOMPUTER TECHNIK MBH, 69469 Weinheim (DE)
(72) Erfinder: Helmle, Herbert, D-69221 Dossenheim (DE); Müller, Klaus, D-69120 Heidelberg (DE)
(74) Vertreter: Meyer-Roedern, Giso, Dr.
(86) Internationale Anmeldenummer: DE9500720
(87) Internationale Veröffentlichungsnummer: WO9533402

(56) Entgegenhaltungen:
- WO-A-86/04799
- WO-A-92/05736
- US-A- 4 762 410
- US-A- 4 850 691
- US-A- 5 214 455

## Beschreibung

Die Erfindung betrifft eine Vorrichtung zum Messen der Pupillenreaktion eines Auges mit wenigstens einer das Auge beleuchtenden Infrarotlichtquelle, mit einem Infrarotdetektor hoher Zeitauflösung, der von dem Auge reflektiertes Infrarotlicht empfängt und ein der anstehenden Lichtintensität entsprechendes Signal abgibt, dessen zeitlicher Verlauf erfaßbar und auswertbar ist, und mit einer Einrichtung zum Erzeugen eines Reizes. Eine gattungsgemäße Vorrichtung ist aus WO-A-92/05736 bekannt.

Die Beobachtung des Pupillenreflexes, insbesondere die Erfassung der zeitlichen Änderung der Pupillengröße auf einen äußeren Reiz, ist von klinischer Bedeutung, da Schlüsse auf den Funktionszustand der an der Reizleitung beteiligten Nervenbahnen und Gehirnteile gezogen werden können. Da nicht nur optische, sondern auch akustische, taktile, thermische u. a. Reize in Betracht kommen, läßt sich eine recht große Zahl von Nervenbahnen prüfen.

Bei der reflektionsphotometrischen Messung einer Änderung der Pupillengröße mit Infrarotlicht nutzt man aus, daß die Iris Infrarotlicht gut reflektiert, der durch die Pupillenöffnung sichtbare Augenhintergrund aber nicht. Eine Pupillenreaktion führt daher zu einer Änderung des vom Auge reflektierten Infrarotlichtpegels entsprechend dem sich ändernden Flächenverhältnis von Iris und Pupillenöffnung.

Aus der DE 25 36 801 A1 ist eine Vorrichtung der eingangs genannten Art bekannt, bei der die Meßoptik an einer Kontaktlinse angeordnet ist. Die Infrarotlichtquelle, der Infrarotdetektor und eine Lichtquelle zum optischen Reiz des Auges liegen dem Auge an der Rückseite der Kontaktlinse gegenüber. Es ist daher nicht möglich, die Pupille beim Aufsetzen der Kontaktlinse, geschweige denn während der Messung visuell zu beobachten.

Die DE 22 11 354 A1 beschreibt eine Vorrichtung, bei der das Auge während einer reflektionsphotometrischen Messung der Pupillenreaktion mit sichtbarem Licht durch einen teildurchlässigen Spiegel beobachtet werden kann. Bei einer Infrarotlichtmessung besteht eine vergleichbare Beobachtungsmöglichkeit nicht. Eine Messung der Pupillenreaktion mit sichtbarem Licht bringt das Problem mit sich, daß die Versuchsperson geblendet und der Pupillenreflex in unerwünschter Weise beeinflußt werden kann.

Nach dem Stand der Technik ist auch eine Videoerfassung der Pupillenreaktion im optischen Spektralbereich mit anschließender Bilddatenverarbeitung bekannt. Dieses Verfahren ist in der Zeitauflosung durch die Videobildfrequenz begrenzt, ermöglicht aber eher als das reflektionsphotometrische Verfahren eine Absolutmessung der Pupillengröße. Die Signalauswertung ist allerdings recht aufwendig.

Aufgabe der Erfindung ist es, eine Vorrichtung der eingangs genannten Art zu schaffen, die ein Ausrichten auf die Pupille und ein infrarotreflektionsphotometrisches Messen der Pupillenreaktion unter visueller Kontrolle des untersuchten Auges ermöglicht.

Diese Aufgabe wird mit einer Vorrichtung der eingangs genannten Art dadurch gelöst, daß in dem Strahlengang des reflektierten Infrarotlichts ein Strahlteiler liegt, der einen Teil des Lichts auf einen Infrarotbildwandler mit einem optischen Monitor gelangen läßt.

Die erfindungsgemäße Infrarotbilderfassung des untersuchten Auges ermöglicht es, die Vorrichtung einfach und genau auf die Pupille auszurichten, Lidschläge und Augenbewegungen zu erkennen und damit einhergehende Meßfehler zu vermeiden. Bei bekanntem Abbildungsmaßstab kann die Infrarotbildinformation zu einer Absolutmessung der Pupillengröße bzw. ihrer Änderung herangezogen werden. Das ermöglicht eine Eichung des in der Zeitauflösung nicht begrenzten reflektionsphotometrischen Analogsignals.

Als Strahlteiler kann zwischen dem Auge und dem Infrarotbildwandler ein für Infrarotlicht teildurchlässiger Spiegel angeordnet sein. Das derart, daß der Infrarotbildwandler durch den Spiegel transmittiertes Infrarotlicht und der Detektor von dem Spiegel reflektiertes Infrarotlicht empfängt, aber auch umgekehrt derart, daß der Detektor durch den Spiegel transmittiertes Infrarotlicht und der Infrarotbildwandler von dem Spiegel reflektiertes Infrarotlicht empfängt. Beide Anordnungen zeichnen sich durch einen einfachen, einzelteilarmen optischen Aufbau aus.

Vorzugsweise schließt der Spiegel mit der Meßachse einen Winkel von 45° ein. Die resultierende 90°-Umlenkung des reflektierten Infrarotlichts ist für eine effektive Streulichtunterdrückung am Detektor von Vorteil.

In einer bevorzugten Bauform liegt der Infrarotbildwandler dem Auge auf der Meßachse gegenüber.

Die Infrarotbilderfassung und -wandlung erfolgt vorzugsweise mit einer CCD-Kamera. Dank der hohen Empfindlichkeit des CCD-Arrays genügt hierfür ein minimaler Intensitätsanteil des reflektierten Infrarotlichts.

Bei einer bevorzugten Bauform ist in einer Beleuchtungsebene durch die Meßachse beidseits davon je eine Infrarotlichtquelle angeordnet. Die Doppelanordnung von Infrarotlichtquellen gewährleistet eine gute Ausleuchtung des untersuchten Auges. Der Spiegel schließt einen Winkel von 45° mit der Beleuchtungsebene ein. Der Detektor ist mit Blickrichtung senkrecht zu der Beleuchtungsebene über der Meßachse angeordnet. Die 90°-Strahlumlenkung zum Detektor ist, wie schon erwähnt, zur effektiven Streulichtunterdrückung von Vorteil.

Für einen optischen Reiz kann eine Blitzlichtquelle vorgesehen sein. Die Lichtintensität der Blitzlichtquelle läßt sich vorzugsweise regeln. Das erlaubt es, die Blitzlichtintensität jeweils so niedrig wie möglich einzustellen und höchst signifikante Pupillenreflexe zu erzeugen. Die Belastung der Versuchsperson ist minimal. Auch kann durch Variieren der Reizlichtintensität das Schwarzweißsehen oder Farbsehen der Versuchsperson gezielt untersucht werden.

Die erfindungsgemäße Vorrichtung ermöglicht sowohl die Untersuchung des nicht konsensuellen Pupillenreflexes, bei der ein und dasselbe Auge stimuliert und beobachtet wird, als auch die Untersuchung des konsensuellen Pupillenreflexes, bei der ein Auge der Versuchsperson stimuliert und das andere beobachtet wird. Für die Untersuchung des nicht konsensuellen Pupillenreflexes hat die Blitzlichtquelle eine bevorzugte Anordnung auf der dem Detektor abgewandten Seite der Beleuchtungsebene unter der Meßachse. Die Abstrahlrichtung der Infrarotlichtquelle(n) und/oder der Blitzlichtquelle ist vorzugsweise gegen die Meßachse geneigt.

Bei einer bevorzugten Ausführungsform ist der Signalausgang des Infrarotdetektors mit einer Sample-and-Hold-Schaltung verbunden. Diese ist geeignet, Störungen des die Pupillenreaktion wiedergebenden zeitabhängigen Intensitätssignals zu eliminieren, die von Augen- und anderen Bewegungen der Versuchsperson, Änderungen der Umgebungshelligkeit o. ä. herrühren. Das ist wegen der verglichen mit solchen Störungen geringen Dynamik des gemessenen Intensitätssignals von Bedeutung.

Vorzugsweise ist der Signalausgang des Infrarotdetektors parallel zu dem Eingang der Sample-and-Hold-Schaltung mit dem einen Eingang eines Operationsverstärkers, und der Ausgang der Sample-and-Hold-Schaltung mit dem anderen Eingang des Operationsverstärkers verbunden. Die Sample-and-Hold-Schaltung läßt sich im Bereitstand auf "Sample" und für den Meßvorgang auf "Hold" schalten. Der Operationsverstärker hat dadurch unabhängig von einem eventuellen Signaloffset des Fotodetektors im Bereitstand einen Ausgangssignalpegel Null. Mit dem durch den Operationsverstärker von Null ansteigend verstärkten Meßsignal nutzt man die volle Dynamik eines nachgeschalteten Analog-Digital-Wandlers aus.

Die erfindungsgemäße Vorrichtung ist vorzugsweise kontaktfrei zum Auge. Das Auge braucht dann für die Untersuchung nicht anästhesiert zu werden, und es wird in keiner Weise mechanisch belastet. Vorzugsweise ist die Vorrichtung ein kompaktes Handgerät, dessen Gehäuse ein Fenster an der dem Auge zugewandten Vorderseite hat. Die Meßperson hält die Vorrichtung entweder frei in der Hand, oder sie legt die die Vorrichtung haltende Hand an den Kopf des Probanden an. Der Monitor für die visuelle Beobachtung des Auges befindet sich vorzugsweise an der dem Auge gegenüberliegenden Rückseite des Gehäuses.

In einer bevorzugten Bauform hat die Infrarotoptik eine geringe Tiefenschärfe, so daß das Monitorbild der Pupille nur bei einem wohldefinierten Meßabstand scharf ist. Die Meßperson kann so das Einhalten des Meßabstands anhand des Monitorbilds kontrollieren.

Ein wohldefinierter Meßabstand ist für eine Absolutmessung der Pupillengröße von Bedeutung. Der Abbildungsmaßstab der Infrarotoptik liegt dann fest, und es kann das Monitorbild der Pupille für die Absolutmessung herangezogen werden. Hierzu empfiehlt es sich, das Zeilensignal wenigstens einer Monitorzeile auszukoppeln und abzuspeichern. Der Pegel des Zeilensignals zeigt am Pupillenrand einen Sprung, der es erlaubt, Pupillenrandpunkte zu diskriminieren und damit nach dem Prinzip der DE 35 41 726 A1 den Durchmesser des Pupillenkreises zu bestimmen.

Die Erfindung wird im folgenden anhand eines in der Zeichnung dargestellten Ausführungsbeispiels näher erläutert. Es zeigen:
- Fig.: 1 die Seitenansicht einer Vorrichtung zum Messen der Pupillenreaktion eines Auges mit teilweise aufgebrochenem Gehäuse;

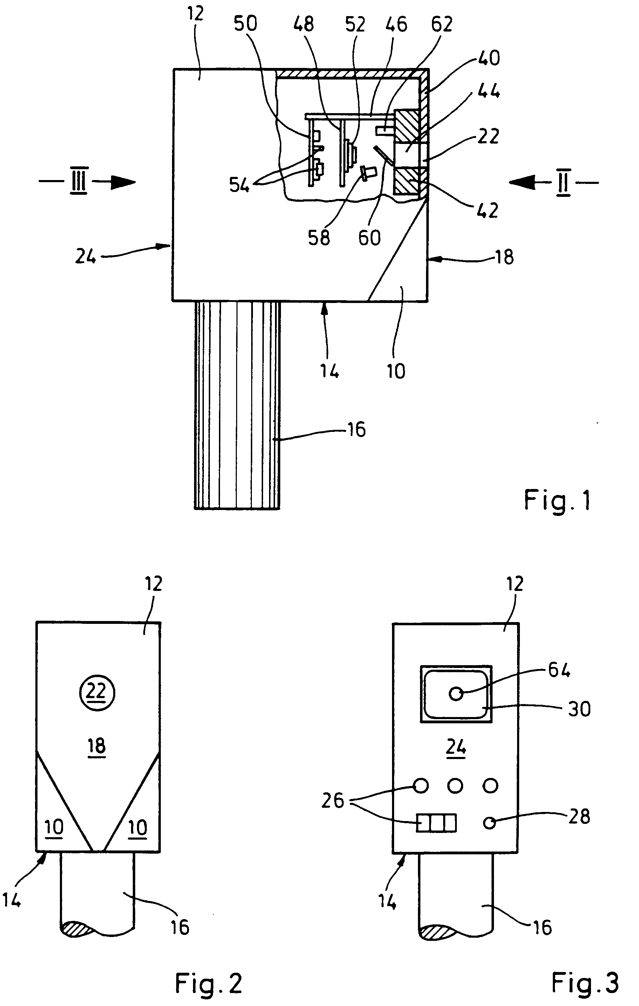

- Fig. 2: einen Blick auf die Vorderseite der Vorrichtung in Richtung II von Fig. 1;
- Fig. 3: einen Blick auf die Rückseite der Vorrichtung in Richtung III von Fig. 1;

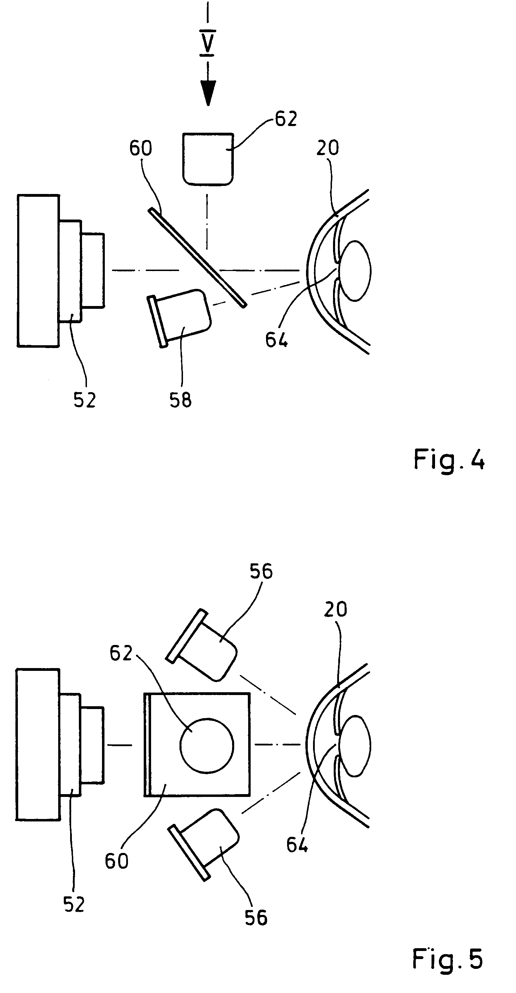

- Fig. 4: schematisch einen Teil der Vorrichtungsoptik in einer Fig. 1 entsprechenden Seitenansicht, wobei zwei in einer Beleuchtungsebene senkrecht zu der Zeichenebene liegende Infrarotlichtquellen nicht dargestellt sind;
- Fig. 5: eine Draufsicht auf die Beleuchtungsebene mit Blick in Richtung V von Fig. 4; und
- Fig. 6: einen Schaltplan.

Die Vorrichtung hat ein im wesentlichen quaderförmiges, an der Unterseite beidseits abgeschrägtes 10 Gehäuse 12. Im rückwärtigen Bereich des Gehäuses 12 ist ein an dessen Unterseite 14 ansetzender Handgriff 16 in Form einer Rundstange vorgesehen, die in der Gehäusemittelebene nach unten ragt. An der Vorderseite 18 des Gehäuses 12 befindet sich ein dem untersuchten Auge 20 bei der Messung gegenüberliegendes Fenster 22. An der Rückseite 24 des Gehäuses 12 sind Bedienungselemente 26, Kontrollampen 28 und ein Monitor 30 zur visuellen Beobachtung des Auges 20 während der Messung angeordnet.

Hinter einer Vorderwand 40 des Gehäuses 12 sitzt eine Platte 42, die Teile der optischen und elektronischen Komponenten der Vorrichtung trägt. Die Platte 42 läßt die Fensteröffnung 22 frei. Sie kann eine mit der Fensteröffnung 22 fluchtende Öffnung 44 haben. Durch die Achse der Öffnung(en) 22, 44 ist eine Meßachse der Vorrichtung definiert.

Von der Platte 42 gehen zum Gehäuseinneren hin Haltestäbe 46 für zwei Platinen 48, 50 ab, die sich im Abstand parallel zu der Plattenebene erstrecken. Die der Platte 42 benachbarte vordere Platine 48 trägt unter anderem eine CCD-Kamera 52, deren infrarotempfindliches CCD-Array der Fensteröffnung 22 auf der Meßachse mit Abstand gegenüberliegt. Die Elektronik 54, mit der die CCD-Kamera 52 beschaltet ist, verteilt sich auf die vordere und hintere Plantine 48, 50.

Die Vorrichtung hat zwei Infrarotlichtquellen 56, die durch das Fenster 22 auf das zu untersuchende Auge 20 zielen. Die Infrarotlichtquellen 56 sind in einer horizontalen Beleuchtungsebene durch die Meßachse angeordnet. Sie sitzen in symmetrischer Anordnung beidseits der Meßachse an der Platte 42 und haben eine gegen die Meßachse geneigte Abstrahlrichtung.

Bei der Untersuchung des nicht konsensuellen Pupillenreflexes wird das infrarotoptisch beleuchtete Auge 20 selbst optisch gereizt. Für den Reiz (Stimulus) ist unterhalb der Beleuchtungsebene eine Blitzlichtquelle 58 angeordnet, die sichtbares Licht vorzugsweise im gelben Spektralbereich abgibt. Die Blitzlichtquelle 58 sitzt unter der Meßachse an der Platte 42. Ihre Abstrahlrichtung ist gegen die Meßachse geneigt, so daß sie auf das zu untersuchende Auge 20 zielt. Die Lichtintensität der Blitzlichtquelle 58 läßt sich regeln. Bei der Untersuchung des konsensuellen Pupillenreflexes dient eine ähnliche Blitzlichtquelle zum optischen Reiz des einen Auges der Versuchsperson, während das andere Auge infrarotoptisch beobachtet wird (nicht dargestellt).

Das von dem Auge 20 reflektierte Infrarotlicht fällt durch das Fenster 22 wieder in das Gehäuse 12 ein. Zwischen der Fensteröffnung 22 und der CCD-Kamera 52 befindet sich ein an der Platte 42 angeordneter Spiegel 60, der unter einem Winkel von 45° gegen die Beleuchtungsebene geneigt ist. Der Spiegel 66 reflektiert ca. 99 % des zurückfallenden Infrarotlichts nach oben. Hier ist oberhalb der Meßachse ein Infrarotdetektor 62 hoher Zeitauflösung angeordnet, der ein der Intensität des anstehenden Infrarotlichts proportionales Analogsignal abgibt. Der zeitliche Verlauf dieses Signals wird mit hoher Frequenz für die weitere Auswertung digitalisiert und abgespeichert, gegebenenfalls an dem Monitor 30 zur Anzeige gebracht, an einem Schreiber oder Plotter ausgegeben u.a.m.

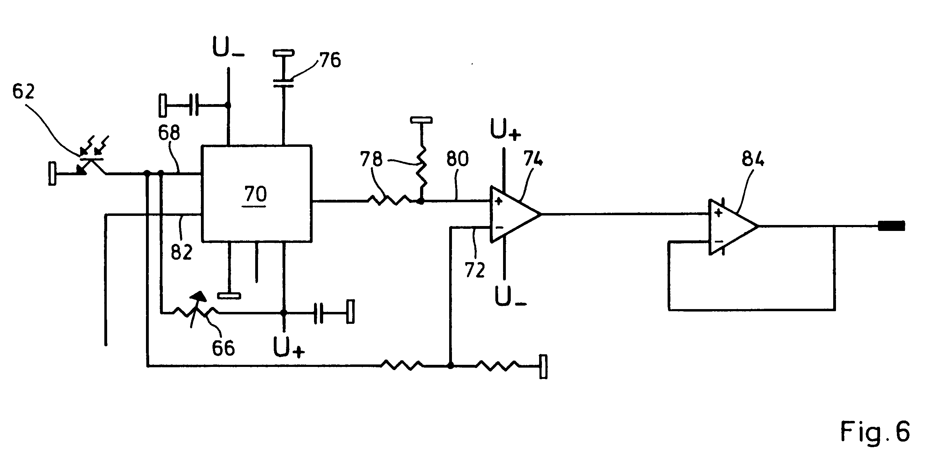

Gemäß Fig. 6 wird dem Ausgangssignal des Infrarotdetektors 62 eine Offsetspannung überlagert, die sich mittels eines veränderlichen Widerstands 66 einstellen läßt, um eine Anpassung an die Hintergrundhelligkeit, Augenfarbe o. ä. vorzunehmen. Das Überlagerungssignal steht an dem Eingang 68 einer Sample-and-Hold-Schaltung 70 und parallel dazu an dem einen Eingang 72 eines Operationsverstärkers 74 an. Die Hold-Kapazität der Sample-and-Hold-Schaltung 70 ist bei 76 angedeutet. Das Ausgangssignal der Sample-and-Hold-Schaltung 70 liegt über eine Spannungsteilerschaltung 78 an dem anderen Eingang 80 des Operationsverstärkers 74 an. Die Sample-and-Hold-Schaltung 70 wird durch ein an dem Eingang 82 anliegendes Steuersignal im Bereitstand auf "Sample" und unmittelbar vor Beginn der Messung einer Pupillenreaktion auf "Hold" geschaltet. Der Operationsverstärker 74 hat dadurch unabhängig von der Offsetspannung im Bereitstand einen Ausgangssignalpegel Null. Das verstärkte Meßsignal wird über eine weitere Verstärkerstufe 84 einem Analog-Digital-Wandler überstellt.

Ca. 1 % des von dem Auge 20 zurückfallenden Infrarotlichts wird durch den Spiegel 60 transmittiert. Es gelangt an die CCD-Kamera 52, die ein Infrarotbild des Auges 20 aufnimmt und in ein sichtbares Bild wandelt. Letzteres kommt an dem Monitor 30 zur Anzeige. Der Benutzer kann dadurch die Vorrichtung genau auf die Pupille 64 des Auges 20 ausrichten. Das wird durch eine in das Monitorbild eingeblendete Zielhilfe erleichtert. Während der Messung sieht der Benutzer, ob das Auge 20 offen oder geschlossen ist. Er kann eventuelle Lidschläge und Augenbewegungen verfolgen und hat nicht zuletzt einen unmittelbaren visuellen Eindruck von der gemessenen Pupillenreaktion.

Die Infrarotoptik hat eine geringe Tiefenschärfe. Das Monitorbild der Pupille ist nur bei Einhaltung des durch den Fokus der Infrarotoptik vorgegebenen Meßabstands scharf. Die Meßperson kann das am Monitor kontrollieren und gegebenenfalls den Meßabstand justieren. Da der Meßabstand und der Abbildungsmaßstab der CCD-Kamera 52 bekannt ist, kann das Monitorbild zu einer Absolutmessung der Pupillengröße herangezogen werden. Man koppelt hierzu das Zeilensignal einer oder mehrerer Monitorzeilen aus und speichert es in geeigneter Weise ab. Eine bestimmte Zeile kann anhand des Vertikalsynchronisationssignals des Monitors herausgegriffen werden. Eine feste Zeilenvorgabe ist ebenso möglich wie eine Zeilenwahl durch die Meßperson. Das Auskoppeln des Zeilensignals wird mit dem Horizontalsynchronisationssignals des Monitors getriggert. Der Pegel des abgespeicherten Zeilensignals zeigt am Pupillenrand eine Sprung, der eine Diskriminierung von Pupillenrandpunkten und damit eine Bestimmung der Größe des Pupillenkreises ermöglicht.

### Liste der Bezugszeichen

- 10: Abschrägung
- 12: Gehäuse
- 14: Unterseite
- 16: Handgriff
- 18: Vorderseite
- 20: Auge
- 22: Fenster
- 24: Rückseite
- 26: Bedienungselement
- 28: Kontrollampe
- 30: Monitor
- 40: Vorderwand
- 42: Platte
- 44: Öffnung
- 46: Haltestab
- 48: vordere Platine
- 50: hintere Platine
- 52: CCD-Kamera
- 54: Elektronik
- 56: Infrarotlichtquelle
- 58: Blitzlichtquelle
- 60: Spiegel
- 62: Infrarotdetektor
- 64: Pupille
- 66: Widerstand
- 68: Eingang
- 70: Sample-and-Hold-Schaltung
- 72: Eingang
- 74: Operationsverstärker
- 76: Hold-Kapazität
- 78: Spannungsteiler
- 80: Eingang
- 82: Eingang
- 84: Verstärker

## Patentansprüche

1. Vorrichtung zum Messen der Pupillenreaktion eines Auges mit wenigstens einer das Auge (20) beleuchtenden Infrarotlichtquelle (56), mit einem Infrarotbildwandler, der von dem Auge reflektiertes Infrarotlicht empfängt und ein Bild des Auges an einem optischen Monitor (30) zur Anzeige bringt, mit einem in dem Strahlengang des reflektierten Infrarotlichts liegenden Strahlteiler und mit einer Einrichtung zum Erzeugen eines Reizes, dadurch gekennzeichnet, daß sie zwei Infrarotdetektoren aufweist, nämlich den Infrarotbildwandler und einen Infrarotdetektor (62) hoher, nicht durch die Videobildfrequenz begrenzter Zeitauflösung, der von dem Auge reflektiertes Infrarotlicht empfängt und ein der anstehenden Lichtintensität entsprechendes Signal abgibt.

2. Vorrichtung nach Anspruch 1, dadurch gekennzeichnet, daß der Strahlteiler ein zwischen dem Auge (20) und dem Infrarotbildwandler liegender, für Infrarotlicht teildurchlässiger Spiegel (60) ist.

3. Vorrichtung nach Anspruch 2, dadurch gekennzeichnet, daß der Infrarotbildwandler durch den Spiegel (60) transmittiertes Infrarotlicht und der Detektor (62) von dem Spiegel (60) reflektiertes Infrarotlicht empfängt.

4. Vorrichtung nach Anspruch 2, dadurch gekennzeichnet, daß der Detektor durch den Spiegel transmittiertes Infrarotlicht und der Infrarotbildwandler von dem Spiegel reflektiertes Infrarotlicht empfängt.

5. Vorrichtung nach einem der Ansprüche 1 bis 4, dadurch gekennzeichnet, daß der Spiegel (60) mit der Meßachse einen Winkel von 45° einschließt.

6. Vorrichtung nach einem der Ansprüche 1 bis 5, dadurch gekennzeichnet, daß der Infrarotbildwandler dem Auge (20) auf der Meßachse gegenüberliegt.

7. Vorrichtung nach einem der Ansprüche 1 bis 6, dadurch gekennzeichnet, daß der Infrarotbildwandler eine CCD-Kamera (52) ist.

8. Vorrichtung nach einem der Ansprüche 1 bis 7, dadurch gekennzeichnet, daß in einer Beleuchtungsebene durch die Meßachse beidseits davon je eine Infrarotlichtquelle (56) angeordnet ist, daß der Spiegel (60) einen Winkel von 45° mit der Beleuchtungsebene einschließt, und daß der Detektor (62) mit Blickrichtung senkrecht zu der Beleuchtungsebene über der Meßachse angeordnet ist.

9. Vorrichtung nach einem der Ansprüche 1 bis 8, dadurch gekennzeichnet, daß sie für einen optischen Reiz des beobachteten oder anderen Auges (20) einer Versuchsperson mit einer in der Lichtintensität vorzugsweise regelbaren Blitzlichtquelle (58) versehen ist.

10. Vorrichtung nach einem der Ansprüche 1 bis 9, dadurch gekennzeichnet, daß die Blitzlichtquelle (58) auf der dem Detektor (62) abgewandten Seite der Beleuchtungsebene unter der Meßachse angeordnet ist.

11. Vorrichtung nach einem der Ansprüche 1 bis 10, dadurch gekennzeichnet, daß die Abstrahlrichtung der Infrarotlichtquelle(n) (56) und/oder der Blitzlichtquelle (58) gegen die Meßachse geneigt ist.

12. Vorrichtung nach einem der Ansprüche 1 bis 11, dadurch gekennzeichnet, daß der Signalausgang des Infrarotdetektors (62) mit einer Sample-and-Hold-Schaltung (70) verbunden ist.

13. Vorrichtung nach Anspruch 12, dadurch gekennzeichnet, daß der Signalausgang des Infrarotdetektors (62) parallel zu dem Eingang (68) der Sample-and-Hold-Schaltung (70) mit dem einen Eingang (72) eines Operationsverstärkers (74) und der Ausgang der Sample-and-Hold-Schaltung (70) mit dem anderen Eingang (80) des Operationsverstärkers (74) verbunden ist, und daß die Sample-and-Hold-Schaltung (70) im Bereitstand auf "Sample" und für den Meßvorgang auf "Hold" schaltbar ist.

14. Vorrichtung nach einem der Ansprüche 1 bis 13, dadurch gekennzeichnet, daß sie ein von Hand zu haltendes Gehäuse (12) mit einem Fenster (22) an der dem Auge (20) zugewandten Vorderseite (18) und einem Monitor (30) vorzugsweise an der gegenüberliegenden Rückseite (24) hat.

15. Vorrichtung nach einem der Ansprüche 1 bis 14, dadurch gekennzeichnet, daß die Infrarotoptik eine geringe Tiefenschärfe hat, so daß das Monitorbild der Pupille nur bei einem wohldefinierten Meßabstand scharf ist.

16. Vorrichtung nach einem der Ansprüche 1 bis 15, dadurch gekennzeichnet, daß das Zeilensignal wenigstens einer Monitorzeile auskoppelbar und abspeicherbar ist.

## Claims

1. Instrument for measuring eye-pupil reaction with at least one infared light source (56) which illuminates the eye (20), with an infrared image converter which receives infrared light reflected by the eye and displays an image of the eye at an optical monitor (30), with a beam splitter located in the path of the reflected infrared light, and with a device for producing a stimulus, characterized in that it comprises two infrared detectors, namely the infrared image converter and an infrared detector (62) of high time resolution not restricted by the video image frequency which receives infrared light reflected by the eye and generates a signal corresponding to the incoming light intensity.

2. Instrument according to Claim 1, characterized in that the beam splitter is a mirror (60) partly transparent for infrared light, arranged between the eye (20) and the infrared image converter.

3. Instrument according to Claim 2, characterized in that the infrared image converter receives infrared light transmitted through the mirror (60), and the detector (62) receives infrared light reflected by the mirror (60).

4. Instrument according to Claim 2, characterized in that the detector receives infrared light transmitted through the mirror and the infrared image converter receives infrared light reflected by the mirror.

5. Instrument according to one of Claims 1 to 4, characterized in that the mirror (60) encloses an angle of 45° with the measurement axis.

6. Instrument according to one of Claims 1 to 5, characterized in that the infrared image converter lies opposite the eye (20) on the measurement axis.

7. Instrument according to one of Claims 1 to 6, characterized in that the infrared image converter is a CCD camera (52).

8. Instrument according to one of Claims 1 to 7, characterized in that an infrared light source (56) is arranged on each of both sides of an illumination plane through the measurement axis, that the mirror (60) encloses an angle of 45° with the illumination plane, and that the detector (62) is arranged with its viewing direction perpendicular to the illumination plane, above the measurement axis.

9. Instrument according to one of Claims 1 to 8, characterized in that for an optical stimulus of the observed eye or the other eye (20) of a person being tested, a flash light source (58), the intensity of which can preferably be regulated, is provided.

10. Instrument according to one of Claims 1 to 9, characterized in that a flash light source (58) is arranged on the side of the illumination plane facing away from the detector (62), below the measurement axis.

11. Instrument according to one of Claims 1 to 10, characterized in that the beam direction of the infrared light source(s) (56) and/or the flash light source (58) is inclined towards the measurement axis.

12. Instrument according to one of Claims 1 to 11, characterized in that the signal output of the infrared detector (62) is connected with a sample-and-hold circuit (70).

13. Instrument according to Claim 12, characterized in that the signal output of the infrared detector (62) is connected with an input (72) of an operation amplifier (74), in parallel with the input (68) of the sample-and-hold circuit (70), and the output of the sample-and-hold circuit (70) is connected with the other input (80) of the operation amplifier (74), and that the sample-and-hold circuit (70) can be switched to "Sample" in the ready state and to "Hold" for the measurement process.

14. Instrument according to one of Claims 1 to 13, characterized in that it has a housing (12), to be held by hand, with a window (22) on the front (18) which faces the eye (20), and a monitor (30) preferably on the back (24), opposite.

15. Instrument according to one of Claims 1 to 14, characterized in that the infrared optics have a low depth of focus, so that the monitor image of the pupil is only clear at a well-defined measurement distance.

16. Instrument according to one of Claims 1 to 15, characterized in that the line signal of at least one monitor cell can be captured and stored.

## Revendications

1. Dispositif de mesure de la réaction d'un oeil au niveau pupillaire, comprenant au moins une source de lumière infrarouge (56) éclairant l'oeil (20), comprenant un convertisseur d'image à infrarouge qui reçoit la lumière infrarouge réfléchie par l'oeil et envoie une image de l'oeil à un moniteur optique (30) pour qu'il l'affiche, comprenant un diviseur de faisceau placé dans la trajectoire de la lumière infrarouge réfléchie, et comprenant un dispositif produisant un stimulus, caractérisé en ce qu'il comporte deux détecteurs d'infrarouges, à savoir le convertisseur d'image à infrarouge et un détecteur d'infrarouges (62) à haut pouvoir de résolution dans le temps, non limité par la fréquence de l'image vidéo, qui reçoit la lumière infrarouge réfléchie par l'oeil et produit un signal correspondant à l'intensité lumineuse existante.

2. Dispositif selon la revendication 1, caractérisé en ce que le diviseur de faisceau est un miroir (60) placé entre l'oeil (20) et le convertisseur d'image à infrarouge, laissant partiellement passer la lumière infrarouge.

3. Dispositif selon la revendication 2, caractérisé en ce que le convertisseur d'image à infrarouge reçoit de la lumière infrarouge transmise par le miroir (60) et le détecteur (62) reçoit de la lumière infrarouge réfléchie par le miroir (60).

4. Dispositif selon la revendication 2, caractérisé en ce que le détecteur reçoit de la lumière infrarouge transmise par le miroir et le convertisseur d'image à infrarouge reçoit de la lumière infrarouge réfléchie par le miroir.

5. Dispositif selon l'une des revendications 1 à 4, caractérisé en ce que le miroir (60) forme, avec l'axe de mesure, un angle de 45°.

6. Dispositif selon l'une des revendications 1 à 5, caractérisé en ce que le convertisseur d'image à infrarouge est situé à l'opposé de l'oeil (20), sur l'axe de mesure.

7. Dispositif selon l'une des revendications 1 à 6, caractérisé en ce que le convertisseur d'image à infrarouge est une caméra à couplage de charge (CCD) (52).

8. Dispositif selon l'une des revendications 1 à 7, caractérisé en ce qu'une source de lumière infrarouge (56) est respectivement placée de part et d'autre de l'axe de mesure dans un plan d'éclairage traversant celui-ci, en ce que le miroir (60) forme un angle de 45° avec le plan d'éclairage, et en ce que le détecteur (62) est placé sur l'axe de mesure, avec sa direction de visée perpendiculaire au plan d'éclairage.

9. Dispositif selon l'une des revendications 1 à 8, caractérisé en ce qu'il est pourvu, pour un stimulus optique de l'oeil observé (20) ou de l'autre oeil d'une personne testée, d'une source de lumière de flash (58) dont l'intensité lumineuse est, de préférence, réglable.

10. Dispositif selon l'une des revendications 1 à 9, caractérisé en ce que la source de lumière de flash (58) est située, du côté du plan d'éclairage qui est opposé au détecteur (62), sous l'axe de mesure.

11. Dispositif selon l'une des revendications 1 à 10, caractérisé en ce que la direction du faisceau de la/des source(s) de lumière infrarouge (56) et/ou de la source de lumière de flash (58) est inclinée en direction de l'axe de mesure.

12. Dispositif selon l'une des revendications 1 à 11, caractérisé en ce que la sortie du signal du détecteur à infrarouge (62) est reliée à un échantillonneur (70).

13. Dispositif selon la revendication 12, caractérisé en ce que la sortie du signal du détecteur d'infrarouges (62), parallèlement à l'entrée (68) de l'échantillonneur (70), est reliée à l'entrée (72) d'un amplificateur opérationnel (74) et la sortie de l'échantillonneur (70) est reliée à l'autre entrée (80) de l'amplificateur opérationnel (74), et en ce que l'échantillonneur (70), en position d'attente, peut être commuté sur "échantillonner" et sur "maintien" pour la procédure de mesure.

14. Dispositif selon l'une des revendications 1 à 13, caractérisé en ce qu'il comporte un boitier (12), destiné à être tenu à la main, avec une fenêtre (22) sur la face avant (18) orientée en direction de l'oeil (20), et avec un moniteur (30), de préférence sur la face arrière (24) opposée.

15. Dispositif selon l'une des revendications 1 à 14, caractérisé en ce que l'optique pour rayons infrarouges a une faible profondeur de champ, de sorte que l'image que donne le moniteur de la pupille n'est nette qu'à une distance de mesure bien définie.

16. Dispositif selon l'une des revendications 1 à 15, caractérisé en ce que le signal transmis par la ligne d'au moins une ligne du moniteur peut être capté et conservé en mémoire.
